Europäisches Patentamt

European Patent Office

Office européen des brevets

(19)

(11) Veröffentlichungsnummer : **0 165 947 B1**

(12) **EUROPÄISCHE PATENTSCHRIFT**

(45) Veröffentlichungstag der Patentschrift :
**10.02.88**

(51) Int. Cl.⁴ : **A 61 F 2/34**

(21) Anmeldenummer : **85900037.4**

(22) Anmeldetag : **11.12.84**

(86) Internationale Anmeldenummer :
**PCT/CH 84/00194**

(87) Internationale Veröffentlichungsnummer :
**WO/8502535 (20.06.85 Gazette 85/14)**

(54) **KÜNSTLICHE GELENKPFANNE.**

(30) Priorität : **16.12.83 CH 6714/83**

(43) Veröffentlichungstag der Anmeldung :
**02.01.86 Patentblatt 86/01**

(45) Bekanntmachung des Hinweises auf die Patenterteilung : **10.02.88 Patentblatt 88/06**

(84) Benannte Vertragsstaaten :
**AT BE CH DE FR GB LI LU NL SE**

(56) Entgegenhaltungen :
**DE-A- 2 823 306**
**DE-A- 3 006 179**
**DE-A- 3 205 526**
**FR-A- 2 310 121**

(73) Patentinhaber : **Protek AG**
**Stadtbachstrasse 64**
**CH-3012 Bern (CH)**

(72) Erfinder : **SUTTER, Franz**
**Bennwilerstrasse 42**
**CH-4435 Niederdorf (CH)**
Erfinder : **MÜLLER, Maurice, E.**
**Melchenbühlweg 9**
**CH-3006 Bern (CH)**
Erfinder : **STRAUMANN, Fritz**
**Hauptstrasse 28**
**CH-4437 Waldenburg (CH)**

(74) Vertreter : **Lusuardi, Werther Giovanni, Dr.**
**Patentanwalt Stockerstrasse 8**
**CH-8002 Zürich (CH)**

EP 0 165 947 B1

**Beschreibung**

Es ist in der Chirurgie bekannt, bei Menschen und Tieren für die Bildung künstlicher Hüftgelenke aus einer im wesentlichen kugelkalottenförmigen Kunststoff-Schale bestehende Gelenkpfannen einzuzementieren. Da diese Gelenkpfannen eine, abgesehen von allenfalls vorhandenen, kleinen Rillen, vollständig kugelkalottenförmige Aussenfläche besitzen, entstehen jedoch bei Schwenkbewegungen der durch derartige künstliche Gelenke miteinander verbundenen Körperteile grosse Scherkräfte zwischen den Gelenkpfannen und dem Knochen. Diese grossen Scherkräfte können eine Lockerung der Gelenkpfannen verursachen, wobei diese Lockerungsgefahr noch dadurch erhöht wird, dass der Knochenzement das natürliche Knochenmaterial schädigt und häufig dessen Abbau verursacht, so dass die künstliche Gelenkpfanne relativ häufig nach einer kürzeren oder längeren Zeitdauer wieder entfernt werden müssen.

Aus der DE-A-2 751 537 ist eine Hüftgelenkprothese bekannt, deren Gelenkpfanne eine Schale und einen koaxial zu deren Rotationssymmetrieachse verlaufenden Dorn mit vollem Querschnitt aufweist. Die Schale und der Dorn bestehen entweder vollständig aus Keramikmaterial oder vorzugsweise aus einem metallischen Kern, der allseitig mit einer Keramikschicht überzogen ist. Diese künstliche Gelenkpfanne soll also durch den an ihrer Schale angeordneten Dorn verankert werden. Da der Dorn einen vollen Querschnitt aufweist, ergibt er im Verhältnis zum Knochenmaterialverlust, der durch das zu seiner Aufnahme erforderliche Loch bedingt ist, nur eine verhältnismässig geringe Verbesserung der Verankerung. Zudem ist bei vielen Patienten bei der tiefsten Stelle der schale, d. h. im Bereich von deren Rotationssymmetrieachse, nur noch ein verhältnismässig dünner Knochensteg vorhanden, so dass der Dorn nahezu keine Verankerung mehr ergibt und zudem, wenn er nicht sehr stark gekürzt wird, auf der der Schale abgewandten Seite aus dem Knochen herausragt.

Im weiteren ist aus der DE-A-3 006 179 eine nicht gattungsgleiche Kugelgelenkkopf-Prothese mit einer Kappe bekannt, bei der eine im Innenraum der Kappe befindliche Hülse als Verankerungsteil dient. Die mit dieser bekannten Prothese erzielten Ergebnisse waren allerdings bisher so enttäuschend, dass der Einsatz einer zylindrischen Hülse als Verankerungsteil zu den Misserfolgen gezählt werden musste. Umfangreiche Versuche haben nun überraschend gezeigt, dass die an einer konvexen Schalenaussenfläche angeordnete zylindrische Hülse als Verankerungselement sich nun doch bewährt.

Schliesslich ist aus der FR-A-2 310 121 eine Gelenkprothese bekannt, welche einen dem Verankerungsteil der erfindungsgemässen Gelenkpfanne ähnlichen perforierten Hohlzylinder aufweist, der jedoch nicht für den direkten Kontakt mit dem Knochen bestimmt ist, sondern durch seine Konstruktion ein Austreten des Knochenzementes aus den Perforationen bewirkt, wodurch sich lokale Abstandszapfen aus Knochenzement ergeben.

Der Erfindung liegt nun die Aufgabe zugrunde, eine ohne Knochenzement einzusetzende künstliche Gelenkpfanne zu schaffen, die mit einem möglichst geringen Knochenmaterialverlust verursachenden Verankerungsteil stabil und dauerhaft in einen Knochen verankert werden kann und die zwischen der Gelenkpfanne und dem Knochen auftretenden grossen Scherkräft dauerhaft aufnehmen kann.

Diese Aufgabe wird durch eine künstliche Gelenkpfanne gelöst, die nach der Erfindung gemäss dem Anspruch 1 ausgebildet ist.

Vorteilhafte Ausgestaltungen der Erfindung gehen aus den abhängigen Ansprüche hervor.

Der Erfindungsgegenstand soll nun anhand in der Zeichnung dargestellter Ausführungsbeispiele erläutert werden. In der Zeichnung zeigt

die Figur 1 einen Schnitt durch Teile von Knochen eines Skeletts und ein künstliches Hüftgelenk,

die Figur 2 eine schaubildliche Ansicht der Gelenkpfanne des in der Figur 1 dargestellten Hüftgelenkes, wobei die Gelenkpfanne in grösserem Massstab und aufgebrochen gezeichnet ist,

die Figur 3 einen Schnitt durch eine Variante einer Gelenkpfanne,

die Figur 4 einen Schnitt durch ein Hüftbein und eine andere Variante einer Gelenkpfanne,

die Figur 5 eine andere, teils in Ansicht, teils aufgebrochen dargestellte Variante eines Verankerungsteils,

die Figur 6 einen Schnitt durch ein Hüftbein und noch eine andere Variante der Gelenkpfanne,

die Figur 7 einen Schnitt durch ein Hüftbein und eine weitere Variante einer Gelenkpfanne,

die Figur 8 einen Schnitt durch eine Variante eines Aussenteils der Schale mit einem Verankerungsteil,

die Figur 9 eine Draufsicht auf einen Ausschnitt des in der Figur 8 dargestellten Schalen-Aussenteils in der in der Figur 8 durch den Pfeil IX bezeichneten Blickrichtung,

die Figur 10 eine schaubildlich Ansicht einer anderen Variante einer Gelenkpfanne,

die Figur 11 einen Schnitt durch einen Teil der in der Figur 10 dargestellten Gelenkpfanne,

die Figur 12 einen Schnitt durch das in der Figur 1 dargestellte Hüftbein sowie einen Fräser zum Fräsen eines kugelkalottenförmigen Loches,

die Figur 13 einen Schnitt durch das Hüftbein mit einer eingesetzten Bohrlehre sowie einen Spiralbohrer,

die Figur 14 einen Schnitt durch das Hüftbein mit einer eingesetzten Fräslehre sowie einen Fräser zum Fräsen einer Ringnut,

die Figur 15 einen Schnitt durch das Hüftbein mit einem Loch zum Aufnehmen der in den Figuren 1 und 2 dargestellten, künstlichen Gelenkpfanne,

die Figur 16 eine schaubildliche Ansicht des proximalen Endes einer Tibia und einer erfindungsgemässen Tibia-Komponente einer Kniegelenkprothese,

die Figur 17 einen antero-posterioren Schnitt durch eine der Kondylenflächen der in Figur 16 dargestellten Tibia-Komponente,

die Figur 18 eine schaubildliche Ansicht einer erfindungsgemässen Patella-Komponente einer Kniegelenkprothese,

die Figur 19 eine schaubildliche Ansicht der glenoidalen Region einer Skapula mit einer erfindungsgemässen Skapula-Komponente einer Schultergelenkprothese und

die Figur 20 eine schaubildliche Ansicht des proximalen Endes einer Ulna und eines Radius mit einer erfindungsgemässen Ulna-Komponente einer Ellbogengelenkprothese.

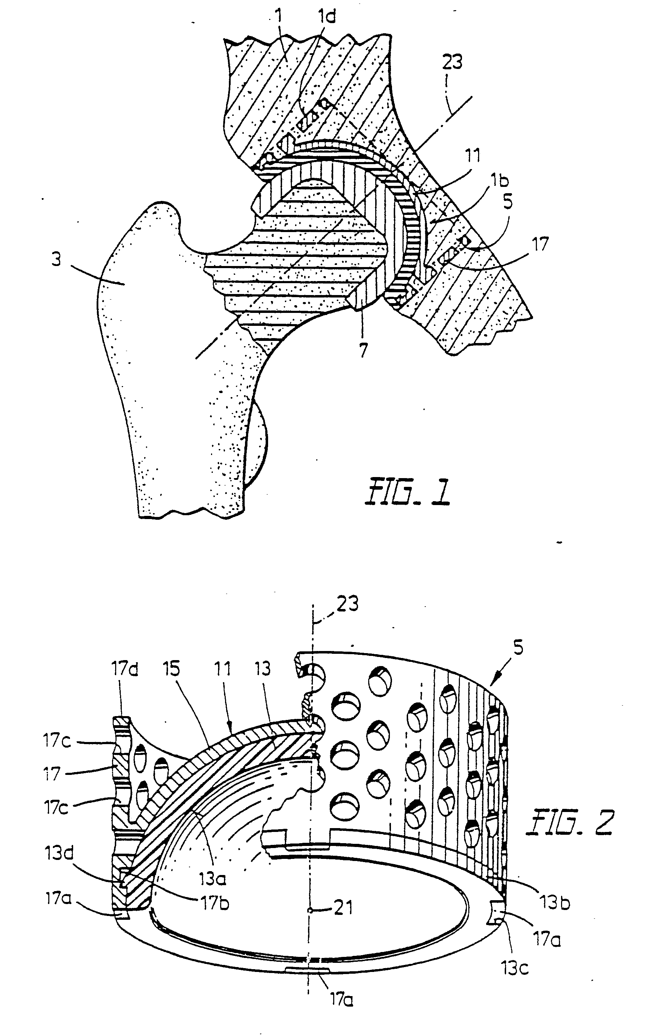

In der Figur 1 ist ein Schnitt durch Teile von zwei Knochen 1, 3, nämlich ein Hüftbein und einen Oberschenkelknochen eines menschlichen Skelettes sowie ein künstliches Hüftgelenk ersichtlich. Das letztere besteht aus einer in das Hüftbein eingesetzten künstlichen Gelenkpfanne 5 und einem am Oberschenkelknochen befestigten künstlichen Gelenkkopf 7 mit einer kugelkalottenförmigen Aussenfläche.

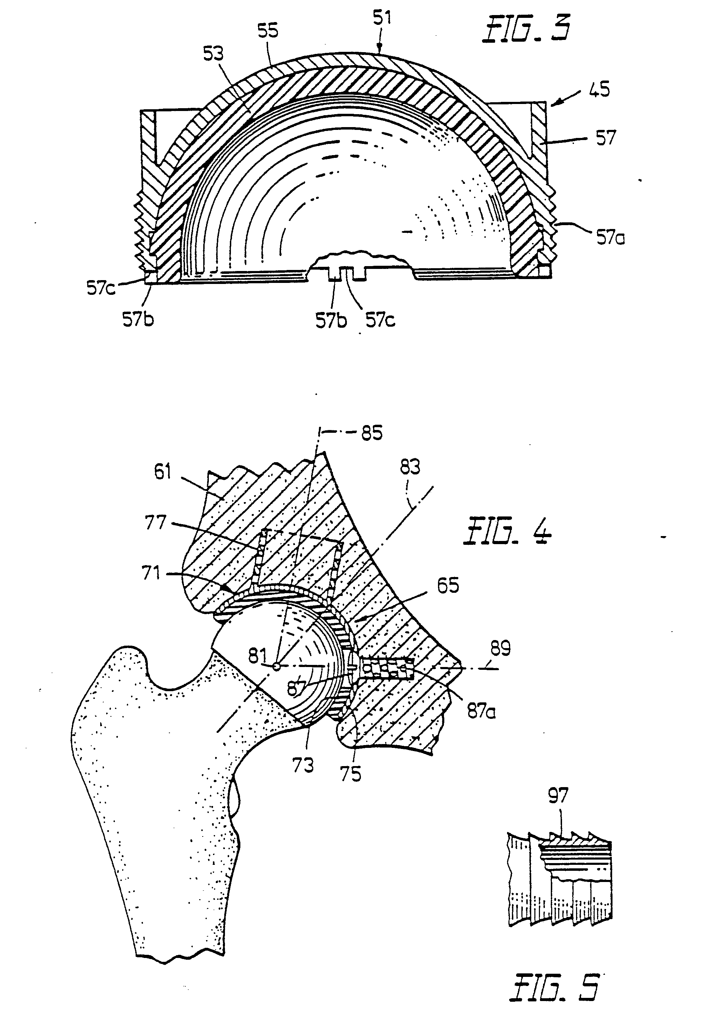

Die separat in der Figur 2 dargestellte Gelenkpfanne 5 weist eine im allgemeinen kugelkalottenförmige Schale 11 auf. Diese ist durch einen Innenteil 13 und einen am Innenteil anliegenden Aussenteil 15 gebildet. Der Innenteil 13 besitzt eine glatte, kugelkalottenförmige Innenfläche 13a die höchstens und ungefähr eine Halbkugel bildet und als Gleitfläche für den künstlichen Gelenkkopf 7 dient. Die Aussenfläche des Innenteils 13 ist zum grössten Teil ebenfalls kugelkalottenförmig, besitzt jedoch bei ihrem Rand einen radial nach aussen vorstehenden, mit einigen über seinen Umfang verteilte Einschnitte 13c versehenen Kragen 13b. In der Nähe des letzteren ist noch eine ebenfalls radial nach aussen vorspringende, ringförmige Rippe 13d vorhanden, die eventuell durch Einschnitte in eine Anzahl bogenförmiger Vorsprünge unterteilt sein kann. Der Kragen 13b besitzt eine zylindrische Umfangsfläche und eine radiale Stirnfläche, die beispielsweise durch eine verrundete Übergangsfläche mit der kugelkalottenförmigen Innenfläche 13a zusammenhängt.

Die Gelenkpfanne 5 weist ferner einen hülsen- oder ringförmigen Verankerungsteil 17 auf. Dieser besteht aus dem gleichen Material wie der Aussenteil 15 der Schale 11 und sein eines Ende hängt in der Nähe des Schalenrandes mit dem Aussenteil zusammen, so dass also der Aussenteil 15 und der Verankerungsteil 17 aus einem einstückigen Körper bestehen und starr sowie unlösbar miteinander verbunden sind. Der Verankerungsteil 17 ist im allgemeinen hohlzylindrisch oder, genauer gesagt, im allgemeinen kreiszylindrisch, wobei sein über die kugelkalottenförmige Aussenfläche des Schalen-Aussenteils 15 herausragender Abschnitt hohl ist. Der Verankerungsteil 17 besitzt also eine kreiszylindrische Aussenfläche und mindestens in seinem freien Endabschnitt auch eine kreiszylindrische Innenfläche. Am der Schale 11 abgewandten, freien Ende ist der Verankerungsteil offen. Der Aussendurchmesser des Verankerungsteils 17 ist gleich dem Aussendurchmesser des Kragens 13b des Schalen-Innenteils 13 und der Verankerungsteil 17 besitzt bei seinem mit dem Schalen-Aussenteil 15 zusammenhängenden Rand axial vorstehende Nocken 17a, die satt in die Einschnitte 13c eingreifen. Der Verankerungsteil 17 weist ferner bei seinem mit dem Aussenteil 15 zusammenhängenden Abschnitt eine ringförmige Nut 17b auf, in die die Rippe 13d eingreift. Die Rippe 13d und die Nut 17b verbinden also den Schalen-Innenteil 13 und den aus dem Schalen-Aussenteil 15 sowie dem Verankerungsteil 17 bestehenden Körper axial unverschiebbar miteinander. Ferner bilden die Einschnitte 13c und Nocken 17a Drehsicherungsmittel, die den Schalen-Innenteil 13 und den aus dem Schalen-Aussenteil 15 und dem Verankerungsteil 17 bestehenden Körper drehfest miteinander verbinden. Der letztere und der Schalen-Innenteil 13 sind also mindestens im wesentlichen starr miteinander verbunden.

Der Verankerungsteil 17 kann mit einer Perforation, d. h. mit über seinen Umfang und seine axiale Ausdehnung verteilten, seinen Mantel durchdringenden Löchern 17c versehen sein, die beispielsweise durch Bohrungen gebildet sind.

Die kugelkalottenförmige Innenfläche 13a bildet einen Teil einer Kugelfläche und spannt also eine sich an sie anschmiegende Kugel auf, deren Zentrum mit 21 bezeichnet ist. Im übrigen spannt der Rand der Schale 11 eine Ebene auf. Ferner ist sowohl die Schale 11 als auch der Verankerungsteil 17 im allgemeinen, d. h. wenn man von den Einschnitten 13c, Nocken 17a und den Löchern 17c absieht, rotationssymmetrisch zu einer gemeinsamen Rotationssymmetrieachse 23, die natürlich durch das Kugel-Zentrum 21 verläuft. Der Verankerungsteil 17 ist also, mit anderen Worten gesagt, symmetrisch zur Aussenfläche der Schale an dieser angeordnet.

Der Aussendurchmesser des Verankerungsteils 17 ist gleich dem grössten Aussendurchmesser der ganzen Gelenkpfanne 5 und zudem ungefähr gleich dem Durchmesser der von der Aussenfläche des Schalen-Aussenteils 15 aufgespannten Kugelfläche. Mit anderen Worten gesagt, schmiegt sich der Verankerungsteil 17 beim Rand der Schale 11 ungefähr tangential an diese an und der von der Aussenfläche des Verankerungsteils 17 aufgespannte Zylinder umschliesst die Innenfläche 13a des Schalen-Innenteils 13. Der Innendurchmesser des Mantels des Verankerungsteils 17 beträgt mindestens ein Drittel und vorzugsweise mindestens zwei Drittel des Durchmessers der von der Innenfläche 13a aufgespannten Kugel und ist beispielsweise ungefähr oder mindestens gleich diesem Durchmesser. Die Wanddicke des Mantels des Verankerungsteils 17 ist höchstens 10 % und vorzugsweise höchstens 5 % des Innendurchmessers des Verankerungsteils.

In axialer Richtung ist der Verankerungsteil 17 derart bemessen, dass er mit seinem dem Schalenrand abgewandten, freien Rand 17d höchstens 10 % des Kugel-Radius der kugelkalottenförmigen Innenfläche 13a der Schale 11 über diese herausragt. Vorzugsweise ragt der genannte Rand 17d in axialer Richtung überhaupt nicht über die Schale 11 hinaus, so dass sich also der Verankerungsteil 17 von seiner Verbindungsstelle mit dem Aussenteil 15 in axialer Richtung höchstens oder ungefähr bis zum Schnittpunkt der Rotationssymmetrieachse 23 mit der Aussenfläche des Schalen-Aussenteils 15 erstreckt.

Die Gelenkpfanne besteht aus biokompatiblen Materialien. Der Innenteil 13 der Schale 11 besteht vorzugsweise aus Kunststoff, beispielsweise Polyäthylen. Der Schalen-Aussenteil 15 und der Verankerungsteil 17 bestehen aus Metall, beispielsweise aus Titan, dessen am Knochen anliegende Flächen eventuell mit einer porösen, durch ein Plasmasprühverfahren aufgebrachten Titanschicht versehen sein können. Der aus dem Schalen-Aussenteil 15 und dem Verankerungsteil 17 bestehende, metallische Körper ist abgesehen von einer gewissen Elastizität im wesentlichen starr und formfest. Der aus Kunststoff bestehende Schalen-Innenteil 13 ist ebenfalls ziemlich starr, aber doch noch ausreichend elastisch deformierbar, dass er bei der Herstellung der Gelenkpfanne in den Aussenteil 15 hineingedrückt werden kann und dabei derart vorübergehend elastisch deformiert wird, dass seine Rippe 13d in die Nut 17b einrastet. Die Rippe 13d und die Nut 17b bilden also Rastmittel, die den Schalen-Innenteil 13 mit den restlichen Teilen der Gelenkpfanne lösbar verbinden, oder genauer gesagt, mindestens ermöglichen, die Gelenkpfanne 5 aus zwei ursprünglich separaten Körpern, nämlich eben dem Schalen-Innenteil 13 und dem mit der Hülse 17 zusammenhängenden Schalen-Aussenteil 15 zusammenzusetzen.

Wenn eine künstliche Gelenkpfanne 5 in den Knochen 1 eingesetzt werden soll, wird bei einer chirurgischen Operation der Knochen 1 derart bearbeitet, dass eine kugelkalottenförmige Vertiefung zum Aufnehmen der Schale 11 und eine Ringnut zum Aufnehmen des Verankerungsteil 17 entsteht. Dabei wird die zum Aufnehmen des Verankerungsteil 17 dienende Ringut derart hergestellt und bemessen, dass der Verankerungsteil 17 satt hineinpasst und vorzugsweise beim Einsetzen leicht eingepresst werden muss. Die Gelenkpfanne 5 kann dann als Ganzes zementfrei in den Knochen 1 eingesetzt werden und sitzt schon unmittelbar nach dem Einsetzen einigermassen fest im Knochen 1. Da das Knochenmaterial unmittelbar an den metallischen Flächen des Schalen-Aussenteils 15 und des Verankerungsteils 17 anliegt sowie im Laufe der Zeit gewissermassen an diese anwächst und zudem auch noch in der in der Figur 1 dargestellten Weise durch die Löcher 17c hindurchwächst, ergibt sich eine stabile und dauerhafte Verankerung der künstlichen Gelenkpfanne 5 im Knochen 1.

Der Verankerungsteil 17 ergibt eine beträchtliche Vergrösserung der für die Kraftübertragung zwischen Knochen und Gelenkpfanne wirksamen Flächen. Dabei trägt der Verankerungsteil 17 insbesondere zur Übertragung derjenigen, häufig auftretenden Kräfte bei, die entstehen, wenn der Gelenkkopf eine Schwenkbewegung um eine Achse ausführt, die ungefähr horizontal und ungefähr rechtwinklig zur Rotationssymmetrie 23 durch das Kugel-Zentrum 21 verläuft. Ein anderer wesentlicher Vorteil des als Verankerungsteil der Gelenkpfanne dienenden Verankerungsteils 17 besteht darin, dass wegen seines zentralen Hohlraums zu seiner Aufnahme nur verhältnismässig wenig Knochenmaterial entfernt werden muss, so dass insbesondere auch eine gute Blutversorgung aller an der künstlichen Gelenkpfanne anliegenden Knochenteile ermöglich wird. Wenn der Verankerungsteil 17, wie aus der Figur 1 ersichtlich, höchstens oder ungefähr gleich tief in den Knochen 1 hineinragt wie die Schale 11, kann die Gelenkpfanne auch noch in Knochen vollständig verankert werden, die im Bereich der Gelenkpfanne, parallel zur Rotationssymmetrieachse 23 gemessen, nur eine wenig grössere Abmessung haben als die künstliche Gelenkpfanne.

Der mit der künstlichen Gelenkpfanne 5 zusammen ein künstliches Hüftgelenk bildende, künstliche Gelenkkopf 7 kann aus Metall, beispielsweise Titan, bestehen. Wenn ein künstliches Gelenk während längerer Zeit durch einen Patienten benutzt wird, kann unter Umständen der aus Kunststoff bestehende Innenteil 13 der Schale 11 durch Verschleiss abgenutzt werden. In einem solchen Fall kann der Innenteil 13 anlässlich einer Operation vom metallischen Aussenteil 15 der Schale gelöst und zum Ersetzen herausgenommen werden, wobei bei der Demontage des Innenteils 13 nötigenfalls dessen Beschädigung ohne weiters in Kauf genommen werden kann. Danach kann ein neuer Schalen-Innenteil 13 in den nun bereits im Knochen 1 verankerten Teil der Gelenkpfanne hineingedrückt und mit diesem verrastet werden.

Die in der Figur 3 dargestellte, künstliche Gelenkpfanne 45 weist eine Schale 51 mit einem Innenteil 53 und einem Aussenteil 55 sowie einen Verankerungsteil 57 auf und ist ähnlich ausgebildet wie die Gelenkpfanne 5, unterscheidet sich von dieser jedoch dadurch, dass der Verankerungsteil 57 statt einer Perforation mindestens in einem axialen Teilbereich seiner Aussenfläche eine ein Gewinde 57a bildende Rippe aufweist, wobei das Gewinde selbstschneidend ausgebildet sein kann. Der Verankerungsteil 57 ist wiederum im allgemeinen, d. h. abgesehen von der schraubenlinienförmigen Rippe, zylindrisch und insbesondere am freien Ende hohlzylindrisch sowie offen. Der Schalen-Innenteil 53 ist analog wie der Schalen-Innenteil 13 mit einem Einschnitte aufweisenden Kragen versehen und der Verankerungsteil 57 hat bei jedem Einschnitt ein Paar in diesen hineinragende Nocken 57b, die durch einen Einschnitt 57c voneinander getrennt sind. Der Verankerungsteil 57 ist im Vergleich zum Durchmesser der Schale etwas kürzer als bei der Gelenkpfanne 5, so dass er sich in axialer Richtung also nicht ganz bis zur Stelle erstreckt, bei der die Rotationssymmetrieachse des Verankerungsteils 57 und der Schale 51 die Aussenfläche des Schalen-Aussenteils durchdringt. Der Verankerungsteil 57 könnte jedoch auch bis zur Durchdringungsstelle der Rotationssymmetrieachse durch die Schalen-Aussenfläche verlängert werden. Beim Einsetzen der Gelenkpfanne 45 in einen Knochen kann der Verankerungsteil 57 mit einem in die Einschnitte 57c eingreifenden Schlüssel in den Knochen eingeschraubt werden, was schon unmittelbar nach dem Einsetzen eine gute Verankerung ergibt. Da der Verankerungsteil 57 bei dieser Variante keine Perforations-Löcher aufweist, die vom Knochen durchwachsen werden, kann man die Gelenkpfanne 45 später relativ einfach aus dem Knochen herausschrauben, falls dies aus irgend einem Grund nötig sein sollte.

In der Figur 4 ist ein Knochen 61 ersichtlich, in dem eine künstliche Gelenkpfanne 65 mit einer einen Innenteil 73 sowie einen Aussenteil 75 aufweisenden Schale 71 und einem beispielsweise perforierten, im allgemeinen hohlzylindrischen Verankerungsteil 77 eingesetzt ist, wobei der Schalen-Innenteil und -Aussenteil analog miteinander verbunden sind wie bei den Gelenkpfannen 5 und 45. Die Schale 71 ist im allgemeinen zu einer Rotationssymmetrieachse 83 rotationssymmetrisch und der Verankerungsteil 77 ist im allgemeinen zu einer Rotationssymmetrieachse 85 rotationssymmetrisch. Die beiden Rotationssymmetrieachsen 83, 85 verlaufen beide durch das Zentrum 81 der von der Innenfläche der Schale aufgespannten Kugel, bilden jedoch miteinander einen beispielsweise 20° bis 45° betragenden Winkel, so dass der Verankerungsteil 77 also asymmetrisch zur Aussenfläche der Schale an dieser angeordnet ist. Bei dieser Variante ist der Verankerungsteil im Vergleich zum Durchmesser der Schale dünner als bei den Gelenkpfannen 5 und 45. Der Innendurchmesser des Verankerungsteils 77 kann jedoch immer noch mindestens 20 % und sogar ohne weiteres mindestens ein Drittel des Durchmessers der von der Schalen-Innenfläche definierten Kugel betragen. Im übrigen kann die Schale 71 noch bei einer bezüglich des Verankerungsteils 77 versetzten Stelle ein Schraubenloch aufweisen, in welchem eine einen zweiten Verankerungsteil 87 bildende Schraube mit versenktem Kopf angeordnet und in den Knochen 61 eingeschraubt ist. Der mit einem Schlitz versehene Schraubenkopf liegt dabei auf einer beispielsweise konischen Fläche des metallischen Aussenteils 75 der Schale 71 auf, während der aus Kunststoff bestehende Schalen-Innenteil 73 lediglich ein nichts zum Tragen der Schraube beitragendes Durchgangsloch für einen Schraubenzieher aufweist. Es sei hierbei vermerkt, dass der Schraubenkopf statt eines Schlitzes natürlich auch ein Sechskantloch oder dergleichen aufweisen könnte. Der zweite Verankerungsteil 87 ist im allgemeinen zu einer Rotationssymmetrieachse 89 rotationssymmetrisch, die zweckmässigerweise ebenfalls durch das Zentrum 81 verläuft. Der Gewindeteil 87a ist vorzugsweise hohl sowie an seinem dem Kopf abgewandten Ende offen und also im allgemeinen ebenfalls hohlzylindrisch, wobei er im übrigen auch noch perforiert sein kann. Der Innendurchmesser des hohlen Gewindeteils 87a dieses zweiten Verankerungsteils 87 beträgt dann allerdings nur ungefähr oder mindestens 10 % des Durchmessers der von der Schalen-Innenfläche aufgespannten Kugel.

Wenn die Gelenkpfanne 65 in den vorgängig bearbeiteten Knochen 61 eingesetzt werden soll, kann man die Gelenkpfanne zuerst in eine Drehstellung bringen, in der die Gelenkpfanne bezüglich ihrer in der Figur 4 dargestellten Lage ungefähr um 180° um die Rotationssymmetrieachse 85 des Verankerungsteils 77 verdreht ist. In dieser Drehstellung kann nun der Verankerungsteil 77 in die im Knochen 61 vorhandene Ringnut hineingedrückt werden, bis die Schale 71 an der kugelkalottenförmigen Vertiefung des Knochens ansteht. Danach kann die Schale 71 um die Rotationssymmetrieachse 85 in die in der Figur 4 gezeichnete Stellung gedreht werden. Schliesslich kann noch der schraubenförmige Verankerungsteil eingeschraubt werden, falls ein solcher vorgesehen ist.

In der Figur 5 ist eine Variante eines Abschnitts eines Verankerungsteils 97 dargestellt, der an seiner Aussenfläche mit mindestens einer und vorzugsweise mehreren, in axialer Richtung gegeneinander versetzten Erhöhungen, nämlich ringförmigen Rippen versehen ist. Diese können im Profil beispielsweise sägezahn- oder widerhakenartig sein, wobei die steileren Rippenflanken dem freien Ende des Verankerungsteils 97 abgewandt sind. Diese Ausbildung des Verankerungsteils 97 ermöglicht, diesen in eine Ringnut eines Knochens einzusetzen, deren Aussendurchmesser kleiner als der grösste Aussendurchmesser der Rippen, d. h. der Rippen-Aussenkanten, und grösser als der Aussendurchmesser der Rippenwurzeln ist. Die Rippen dringen dann schon beim Einsetzen der Gelenkpfanne in den Knochen ein wenig in diesen ein. Der Verankerungsteil 97 ist abgesehen von diesen Rippen wiederum hohlzylindrisch. Ein solcher Verankerungsteil kann ähnlich wie der in der Figur 4 dargestellten Verankerungsteil 77 an der Schale angeordnet und bemessen werden.

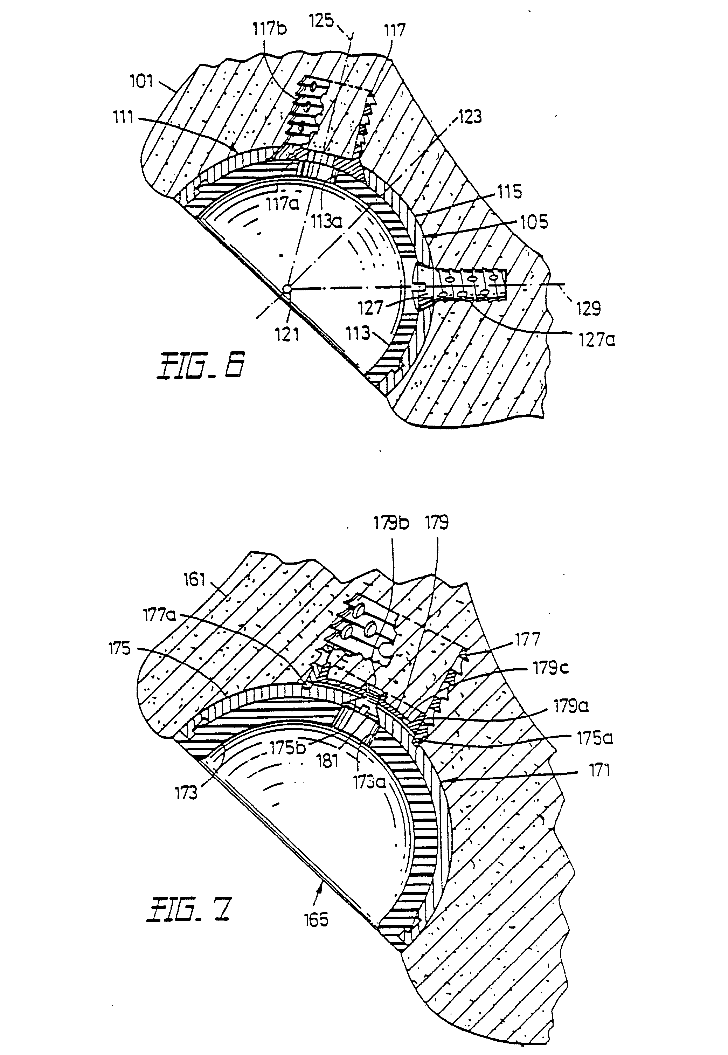

In der Figur 6 ist ein Knochen 101, nämlich ein Hüftbein mit einer künstlichen Gelenkpfanne 105 ersichtlich, die eine Schale 111 mit einem Innenteil 113 aus Kunststoff und einem Aussenteil 115 aus Metall aufweist, wobei der Innenteil 113 und der Aussenteil 115 analog miteinander verbunden sein können wie bei den Gelenkpfannen 5, 45, 65. Der zur Gelenkpfanne 105 gehörende Verankerungsteil 117 ist als hohle, in den Knochen 101 eingeschraubte Schraube ausgebildet und weist einen Kopf 117a und einen hohlen, an seinem dem Kopf 117a abgewandten Ende im allgemeinen zylindrischen, mit mindestens einer ein Aussengewinde bildenden Rippe versehenen und eventuell perforierten Mantel 117b auf.

Der Kopf 117a weist eine sich zum Mantel 117b hin verjüngende, konische oder eventuell eine Kugelzone bildende Umfangsfläche auf, mit der er auf einer ungefähr komplementär ausgebildeten Fläche eines Loches des Schalen-Aussenteils 115 aufliegt. Die Stirnfläche des Kopfs 117a ist konkav gekrümmt und die axiale Abmessung des Kopfs 117a ist derart bemessen, dass er ausserhalb des Schalen-Innenteils 113 im erwähnten Loch des Schalen-Aussenteils 115 Platz findet. Im übrigen ist der Kopf 117a mit Mitteln zum Angreifen mit einem Schlüssel, beispielsweise einem Sechskantloch versehen. Der Schlüssel kann durch ein im Schalen-Innenteil 113 vorhandenes, zum Verankerungsteil 117 koaxiales, vorzugsweise durch eine Bohrung gebildetes Durchgangsloch 113a hindurchgesteckt werden, dessen Durchmesser kleiner als der Aussendurchmesser des Kopfes 117a ist. Der Verankerungsteil 117 ist also nicht starr mit der Schale 111 verbunden, wird jedoch auch dann, wenn die Gelenkpfanne 105 noch nicht in einen Knochen eingesetzt ist, durch den Schalen-Innenteil 113 unverlierbar und eventuell mit etwas Spiel von der Schale 111 gehalten.

Die Schale 111 ist im allgemeinen rotationssymmetrisch zu einer Rotationssymmetrieachse 123 und der Verankerungsteil 117 ist im wesentlichen, d. h. abgesehen vom Sechskantloch seines Kopfes und dem Gewinde sowie der Perforation des Mantels 117b, rotationssymmetrisch zu einer Rotationssymmetrieachse 125, die die Achse 123 im Zentrum 121 der von der Innenfläche des Schalen-Innenteils 113 aufgespannten Kugel schneidet, wobei der Winkel zwischen den beiden Achsen 123, 125 im gleichen Grössenbereich liegen kann wie bei der Gelenkpfanne 65. Der Innendurchmesser des Verankerungsteils 117 beträgt mindestens 20 % und beispielsweise ungefähr oder mindestens 25 % des Durchmessers der von der Innenfläche der Schale 111 aufgespannten Kugel. Ferner kann eventuell noch ein zweiter, durch eine Schraube gebildeter Verankerungsteil 127 vorhanden sein, dessen Gewindeteil 127a hohl, an dem der Schale abgewandten Ende offen und dünner als der Mantel 117b ist und dessen Rotationssymmetrieachse 129 ebenfalls durch das Zentrum 121 verläuft, wobei der Verankerungsteil 127 ähnlich angeordnet sein kann wie der Verankerungsteil 87 der in der Figur 4 dargestellten Gelenkpfanne 65.

Bei der Gelenkpfanne 105 können der Innen- und Aussenteil der Schale 111 und der Verankerungsteil 117 in zusammengebautem Zustand geliefert und als Ganzes gemeinsam in den vorher entsprechend bearbeiteten Knochen 101 eingesetzt werden. Das Einsetzen kann in ähnlicher Weise erfolgen, wie bei der in der Figur 4 dargestellten Gelenkpfanne 65, wobei aber der Verankerungsteil 117 mit einem Inbus-Schlüssel bezüglich der Schale 111 um die Achse 125 gedreht und in die zu seiner Aufnahme im Knochen angebrachte Ringnut eingeschraubt werden kann.

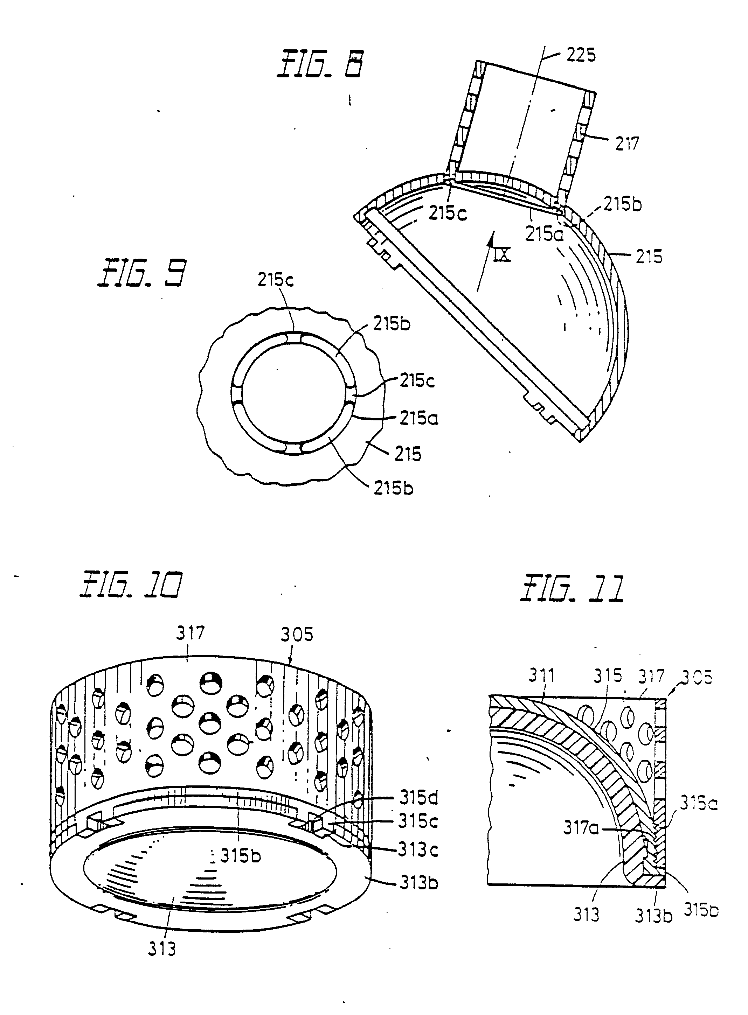

In dem in der Figur 7 dargestellten Knochen 161, nämlich ein Hüftbein, ist eine künstliche Gelenkpfanne 165 eingesetzt. Diese weist eine Schale 171 mit einem Innenteil 173 aus Kunststoff sowie einem Aussenteil 175 aus Metall und einen im allgemeinen hohlzylindrischen Verankerungsteil 177 aus Metall auf. Der Innenteil 173 und der Aussenteil 175 sind ähnlich miteinander verbunden wie bei den vorgängig beschriebenen Gelenkpfannen 5, 45, 65, 105 und der Verankerungsteil 177 ist bezüglich der Schale ähnlich asymmetrisch angeordnet wie bei den Gelenkpfannen 65 und 105. Bei der Gelenkpfanne 165 bestehen der Aussenteil 175 und der Verankerungsteil 177 aus separaten metallischen Werkstücken, wobei der eine Rand des Verankerungsteils 177 in einer Ringnut 175a des Aussenteils sitzt und mit über seinen Umfang verteilten Einschnitten 177a zum Ansetzen eines Einschraubwerkzeuges versehen ist. Der Verankerungsteil 177 ist in der Nähe des genannten Randes ferner mit einem Innengewinde versehen. Ein im Innern des Verankerungsteils 177 angeordneter, metallischer Verbindungsteil 179 weist einen schalenartigen, gewölbten, an der Aussenfläche des Aussenteils 175 anliegenden Hauptabschnitt und einen kragenartigen, vom Aussenteil 175 wegragenden Rand 179a auf, der mit einem Aussengewinde versehen und mit dem erwähnten Innengewinde des Verankerungsteils 177 verschraubt ist, wobei die Einschraubtiefe des Verbindungsteils 179 in den Verankerungsteil 177 zweckmässigerweise durch eine beim Ende von dessen Innengewinde vorhandene Anschlagfläche des letzteren begrenzt und definiert ist. Der Verbindungsteil 179 hat in seinem Zentrum eine zapfenartige Verdickung mit einer Gewindebohrung 179b und ist mit einer in dieser eingeschraubten Schraube 181 lösbar am Aussenteil 175 befestigt, wobei der Kopf der Schraube 181 in einem Schraubenloch 175b des Aussenteils 175 versenkt ist. Der Innenteil 173 kann mit einem mit dem Schraubenloch 175b fluchtenden, durchgehenden Loch 173a versehen sein, durch das hindurch der Schraubenkopf einführbar und zugänglich ist. Ferner ist der Verbindungsteil 179 zweckmässigerweise noch mit um seine Mittelachse herum verteilten Löchern 179c oder Einschnitten zum Angreifen mit einem Schlüssel versehen. Der über die Aussenfläche des Schalen-Aussenteils 173 herausragende Abschnitt des Verankerungsteils 177 ist bei der fertig zusammengebauten Gelenkpfanne 165 wiederum zum grössten Teil, nämlich abgesehen von dem vom Verbindungsteil 179 ausgefüllten Teil seines Innenraumes, hohl und am freien Ende offen.

Vor dem Einsetzen der künstlichen Gelenkpfanne 165 sind deren Schalen-Innenteil 173 und -Aussenteil 175 bereits miteinander verbunden, während der Verankerungsteil 177 und der Verbindungsteil 179 noch von der Schale 171 getrennt aber vorzugsweise miteinander verschraubt sind. Die Schraube 181 ist ebenfalls noch von der Schale getrennt, wobei es jedoch auch möglich wäre, den Durchmesser des Loches 173a etwas kleiner zu bemessen als denjenigen des Schraubenkopfes und die Schraube 181 bereits vor dem Einsetzen der Gelenkpfanne 165 in den Knochen 161 lose aber unverlierbar in der Schale 171 anzuordnen.

Wenn nun die Gelenkpfanne 165 in den vorgängig bearbeiteten Knochen 161 eingesetzt werden soll, schraubt man zuerst den Verankerungsteil 177 zusammen mit dem bereits in diesen eingeschraubten Verbindungsteil 179 in die zu seiner Aufnahme vorgesehen Ringnut des Knochens 161 ein. Dabei kann man zum Einschrauben ein Einschraubwerkzeug mit in die Einschnitte 177a eingreifenden Nocken und einem nach aussen über den Verankerungsteil herausragenden Kragen verwenden. Der letztere ist vorzugsweise mit einer kugelzonenförmigen Anschlagfläche versehen, deren Krümmungsradius gleich dem demjenigen der Aussenfläche des Schalen-Aussenteils 175 ist. Der Verankerungsteil 171 wird dann so tief in den Knochen eingeschraubt, bis die Anschlagfläche des Einschraubwerkzeuges an der kugelkalottenförmigen, zum Aufnehmen der Schale 171 dienenden Vertiefung des Knochens 161 ansteht und der schalenförmige Hauptabschnitt des Verbindungsteils 179 ebenfalls an einer vorgängig bearbeiteten Fläche des Knochens anliegt. Der Verankerungsteil 177 befindet sich dann in der vorgesehenen Tiefe.

Es sei hiebei bemerkt, dass der Verankerungsteil 177 und der Verbindungsteil 179 vor dem Einsetzen in den Knochen noch getrennt sein könnten. In diesem Fall würde man zuerst den Verankerungsteil 177 bis zur vorgesehenen Tiefe in den Knochen einschrauben, bis er an der erwähnten Anschlagfläche des letzteren ansteht. Zum Einschrauben des Verbindungsteils 179 kann dabei ein Schlüssel mit in die Löcher 179c eingreifenden Nocken verwendet werden.

Wenn nun der Verankerungsteil 177 und der Verbindungsteil 179 in den Knochen eingesetzt sind, wird die Schale 171 eingesetzt und mit der Schraube 181 am Verbindungsteil 179 festgeschraubt und dadurch starr aber lösbar mit dem Verankerungsteil 177 verbunden. Im übrigen könnte zusätzlich noch ein durch eine Hohlschraube gebildeter, analog zu dem in der Figur 4 dargestellten Verankerungsteil 87 ausgebildeter und angeordneter Verankerungsteil vorgesehen werden.

Falls es später wegen Abnützungserscheinungen des Schalen-Innenteils 173 erforderlich sein sollte, kann dieser ausgewechselt werden, wobei dies in analoger Weise durchgeführt werden kann, wie es für die Gelenkpfanne 5 beschrieben wurde. Bei der Gelenkpfanne 165 besteht jedoch zusätzlich noch die Möglichkeit, die Schraube 181 loszuschrauben und die ganze Schale 171 zu entfernen, wenn dies wegen einer Infektion oder aus anderen Gründen notwendig werden sollte. Danach kann man noch den Verbindungsteil 179 vom Verankerungsteil 177 losschrauben.

Wenn sich die Gelenkpfanne 165 seit längerer Zeit im Knochen 161 befindet, ist der Knochen fest mit dem Gewinde des Verankerungsteils 177 verwachsen und hat auch dessen Löcher durchwachsen. Falls nun auch noch der Verankerungsteil 177 aus medizinischen Gründen wieder aus dem Knochen herausgenommen werden muss, kann man den Verankerungsteil kaum noch durch blosses Herausschrauben entfernen. Man kann dann jedoch mit zwei Hohlfräsern ausserhalb und innerhalb des Verankerungsteils zu diesem koaxiale Ringnuten in den Knochen fräsen. Danach kann dann der Verankerungsteil herausgezogen werden.

Die künstliche Gelenkpfanne 165 kann also einerseits sehr stabil im Knochen 161 verankert werden und andererseits nötigenfalls trotzdem in verhältnismässig einfacher Weise wieder teilweise oder vollständig aus dem Knochen herausgenommen werden. Dabei ist es von Vorteil, dass auch im Fall, dass eine vollständige Entfernung der Gelenkpfanne notwendig ist, nur verhältnismässig wenig Knochenmaterial entfernt werden muss.

Bei der in den Figuren 8 und 9 dargestellten Variante des metallischen Schalen-Aussenteils 215 ist der hohlzylindrische Verankerungsteil 217 zu einer Rotationssymmetrieachse 225 rotationssymmetrisch, beispielsweise perforiert und bezüglich der Schale ähnlich angeordnet wie bei den in den Figuren 4, 6 und 7 dargestellten Gelenkpfannen 65, 105 und 165. Der in den Figuren 8 und 9 nicht dargestellte Schalen-Innenteil kann ähnlich ausgebildet und mit dem Schalen-Aussenteil 215 verbunden sein, wie bei den vorgängig beschriebenen Ausführungsvarianten. Der Schalen-Aussenteil 215 und der Verankerungsteil 217 hängen unlösbar zusammen, d. h. sie bestehen aus einem einstückigen Körper. Der Aussenteil 215 ist jedoch auf seiner Innenseite mit einer zum Verankerungsteil 225 koaxialen Ringnut 215a versehen. Deren Innendurchmesser ist ungefähr gleich demjenigen des Verankerungsteils 117 oder eventuell etwas kleiner und der Aussendurchmesser der Ringnut 215a ist ungefähr gleich dem Aussendurchmesser des Verankerungsteils 217 oder eventuell ein wenig grösser. Die Ringnut 215a kann in über den Umfang verteilte, abwechselnd aufeinander folgende Abschnitte 215b, 215c unterteilt sein, wobei die Abschnitte 215b länger und tiefer sind als die Abschnitte 215c. Das unter den letzteren vorhandene Material bildet dann gewissermassen über den Grund der Nutabschnitte 215b vorstehende Verstärkungsnocken.

Die den Schalen-Aussenteil 215 und den Verankerungsteil 217 aufweisende Gelenkpfanne wird analog in einen Knochen eingesetzt, wie die in der Figur 4 dargestellte Gelenkpfanne 65. Man kann auch bei einer mit dem Schalen-Aussenteil 215 ausgerüsteten, in einem Knochen verankerten Gelenkpfanne den Schalen-Innenteil vom Aussenteil 215 trennen, falls dies erforderlich ist. Wenn es zudem notwendig werden sollte, auch den Schalen-Aussenteil 215 aus dem Knochen herauszunehmen, kann man den Schalen-Aussenteil bei der Nut 215a vollständig durchfräsen, so dass er in Teile zerfällt, oder eventuell nur die unter den weniger tiefen Nutabschnitten 215c vorhandenen, über die benachbarten, tieferen Nutabschnitte 215b emporragenden Verstärkungsnocken mit einem Fingerfräser wegfräsen und dann den Schalen-Aussenteil 215 vom Verankerungsteil 217 abbrechen, wobei die Ringnut 215a eine Sollbruchstelle bildet. Danach kann man auch den Verankerungsteil 217 aus dem Knochen herausnehmen, wobei letzteres in analoger Weise erfolgen kann wie beim in der Figur 7 dargestellten Verankerungsteil 177.

Die in den Figuren 10 und 11 dargestellte, künstliche Gelenkpfanne 305 weist eine Schale 311 mit einem Innenteil 313 sowie einem Aussenteil 315 und einen hohlzylindrischen, beispielsweise perforierten Verankerungsteil 317 auf, wobei der letztere bezüglich der Schale 311 ähnlich angeordnet ist wie bei der in den Figuren 1 und 2 dargestellten Gelenkpfanne 5, der Verankerungsteil 317 aber lösbar mit dem Schalen-Aussenteil 315 verbunden ist. Der Schalen-Innenteil 313 besitzt ähnlich wie der Schalen-Innenteil 13 bei seinem Rand einen radial nach aussen ragenden Kragen 313b mit Einschnitten 313c. Der Schalen-Aussenteil 315 hat ebenfalls einen radial nach aussen ragenden Kragen 315b, der zwischen dem Kragen 313b und dem einen Rand des Verankerungsteils 317 angeordnet ist, wobei die Umfangsflächen der beiden Krägen 313b, 315b mit der Aussenfläche des Verankerungsteils 317 fluchtend sind. Der Kragen 315b des Schalen-Aussenteils 315 ist für jeden Einschnitt 313c mit einem Paar in diesen hineinragenden, durch einen Einschnitt 315d voneinander getrennten Nocken 315c versehen. Der an den Kragen 315b anschliessende Abschnitt des Schalen-Aussenteils 315 besitzt einen zylindrischen Aussenflächenabschnitt, der mit einem Aussengewinde 315a versehen ist, und der an dem Kragen 315b anstossende Abschnitt des Verankerungsteils 317 ist mit einem Innengewinde 317a versehen, das mit dem Aussengewinde 315a verschraubt ist. Die Gelenkpfanne 305 kann in ähnlicher Weise in einem Knochen verankert werden wie die Gelenkpfanne 5, ermöglicht jedoch die ganze Schale 311 vom Verankerungsteil 317 zu trennen, falls es später notwendig werden sollte, die Gelenkpfanne wieder aus dem Knochen herauszunehmen.

Die Gelenkpfannen können noch in anderer Weise modifiziert werden. Beispielsweise können gewisse Merkmale der verschiedenen, dargestellten Varianten vertauscht oder miteinander kombiniert werden. Dabei wäre es etwa möglich, einen gemäss dem Verankerungsteil 17 in den Figuren 1 und 2 angeordneten und bemessenen Verankerungsteil statt mit einer Perforation oder zusätzlich zu dieser mit mindestens einer Rippe zu versehen, die ein Gewinde bildet oder analog wie in der Figur 5 ringförmig verläuft und eventuell sägezahnartig profiliert ist. Man könnte die Verankerungsteile aber auch mit axialen Rippen oder mit reihenweise angeordneten Zähnen versehen und eventuell zusätzlich perforieren. Andererseits wäre es aber auch möglich, die Mäntel der starr. mit der Schale oder genauer gesagt, mindestens starr mit dem Aussenteil der Schale verbundenen Verankerungsteile glatt zylindrisch und kompakt auszubilden, so dass sie weder eine Perforation noch irgendwelche Rippen aufweisen. Des weitern könnte man eventuell auch den metallischen Aussenteil der Schalen mit durchgehenden Perforationslöchern oder mit Anbohrungen, d. h. Sacklöchern versehen.

Ferner könnte man auch bei denjenigen Varianten, bei denen die Schale und der Verankerungsteil eine gemeinsame Rotationssymmetrieachse aufweisen, den Aussendurchmesser des Verankerungsteils kleiner festlegen als denjenigen der Schale, so dass dann der Verankerungsteil bei einem vom Rand der Schale entfernten, ringförmigen Abschnitt der Schale mit dieser verbunden wäre.

Bei der in der Figur 6 dargestellten Gelenkpfanne 105 könnte man das im Schalen-Innenteil vorhandene zum Verankerungsteil 117 vorhandene Loch auch so gross machen, dass der Kopf 117a des Verankerungsteils hindurchdringen kann. Dies würde ermöglichen, den Verankerungsteil 117 erst in das Loch der Schale einzuführen, wenn diese schon in den Knochen eingesetzt ist. Umgekehrt wäre es es aber auch möglich, die Schalen-Innenteile der in den Figuren 4 und 6 dargestellten Gelenkpfanne 65 und 105 lochfrei auszubilden, so dass die Köpfe der Verankerungsteile 87, 117, 127 durch die Innenteile der Schalen abgedeckt würden. In diesem Fall wären dann der Innen- und der Aussenteil der Schalen vor der Operation noch getrennt und man würde bei der Operation zuerst nur den Schalen-Aussenteil in den Knochen einsetzen, die Verankerungsteile 87 bzw. 117 und 127 im Knochen festschrauben und erst danach den Innenteil der Schale mit dem Aussenteil durch Verrasten verbinden.

Zudem könnte der Rand der Schale zur Anpassung an die Form der Knochenaussenfläche beispielsweise nur in einem Sektor der Schale entlang einer Ebene verlaufen und im restlichen Sektor der Schale gegen diese Ebene geneigt oder gekrümmt sein. Ferner könnte die vom Schalenrand aufgespannte Schmiegefläche sogar überall gekrümmt sein. Eine derartige Schale besitzt dann keine Rotationssymmetrieachse mehr. Wenn der Verankerungsteil der Schale unmittelbar beim Rand der Schale mit dieser zusammenhängt, ist dann auch der Verankerungsteil, streng genommen, nicht mehr vollständig rotationssymmetrisch, wobei aber immerhin noch der nicht unmittelbar mit der Schale zusammenhängende, den Hohlraum umschliessende Mantelabschnitt des Verankerungsteils rotationssymmetrisch zur Achse des Verankerungsteils ist.

Des weitern könnte man die Gelenkpfannen statt aus einem Kunststoff-Metall-Verbundmaterial vollständig aus einem dieser beiden Materialien oder auch ganz oder teilweise aus einem Keramikmaterial herstellen.

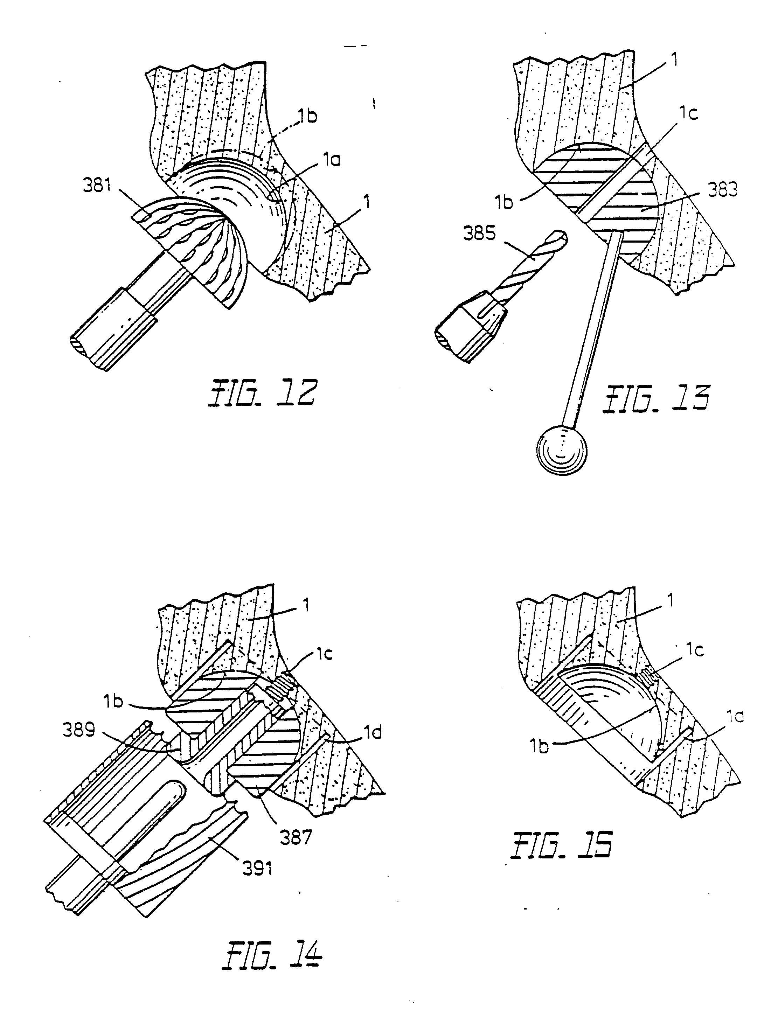

Nun soll anhand der Figuren 12 bis 15 noch die bei einer Operation zum Einsetzen der Gelenkpfanne 5 erforderlich Bearbeitung des Knochens 1, d. h. des Hüftbeins, erläutert werden.

Die Figur 12 zeigt den Knochen 1 mit der noch unbearbeiteten, die natürliche Gelenkpfanne bildenden, ungefähr halbkugelförmigen Vertiefung 1a. Diese natürliche Vertiefung 1a wird nun mit einem eine kugelkalottenförmige und vorzugsweise halbkugelförmige Stirnseite mit Schneiden aufweisenden Fräser 381 ein wenig ausgefräst, nämlich etwas vertieft und/oder im Durchmesser vergrössert, so dass sich die in der Figur 12 strichpunktiert gezeichnete Vertiefung 1b ergibt, die zum grössten Teil halbkugelförmig ist und eventuell bei ihrer Mündung noch einen kurzen zylindrischen Abschnitt aufweist. In die Vertiefung 1b wird nun auf die in der Figur 13 dargestellte Weise die mit einem Handgriff und einer zentralen Führungsbohrung versehene Bohrlehre 383 eingebracht. Mit einem Spiralbohrer 385 wird unter Benutzung der Bohrlehre 383 bei der tiefsten Stelle der Vertiefung eine Bohrung 1c in den Knochen gebohrt, die den Knochen, wie dargestellt, durchdringt, jedoch auch ein Sackloch bilden könnte.

Nun wird der in der Figur 14 dargestellte, eine kugelkalottenförmige Stirnfläche sowie einen zylindrischen Mantelflächenteil und ein koaxiales Durchgangsloch aufweisende, hohle Zapfen 387 in die Vertiefung 1b eingesetzt und mit der einen Kopf sowie ein vorzugsweise selbstschneidendes Gewinde aufweisenden Schraube 389 vorübergehend am Knochen 1 befestigt. Der hohle Zapfen 387 und die in die Bohrung 1c eingeschraubte Schraube 389 bilden zusammen eine Fräslehre für einen als hohlen Stirnfräser ausgebildeten Fräser 391. Der Durchmesser des Hohlraumes des Fräsers 391 ist geringfügig grösser als der Durchmesser des zylindrischen Mantelflächenteils des Zapfens 387, so dass der hohle Fräser mit geringem Spiel über den zylindrischen, aus dem Knochen herausragenden Mantelflächenteil des Zapfens gleiten kann. Die Schraube 389 weist vorzugsweise eine an ihrem kopfseitigen Ende offene, koaxiale Bohrung auf und der Fräser 391 ist dementsprechend vorteilhafterweise in seinem Hohlraum mit einem koaxialen, zylindrischen Dorn versehen, der mit geringem Spiel in die Bohrung der Schraube eindringen kann. Mit dem Fräser 391 wird eine zum Zapfen 387 und zur Schraube 389 koaxiale Ringnut 1d in den Knochen 1 gefräst, wobei der Fräser 391 sowohl durch den zylindrischen Mantelflächenteil des Zapfens 387 als auch durch die Bohrung der Schraube 389 geführt wird. Es sei jedoch bemerkt, dass die Bohrung der Schraube 389 und der zum Eindringen in diese bestimmten Dorn des Fräsers 391 auch wegfallen könnten. Ferner könnte man eventuell die Schraube 389 weglassen und dafür den Zapfen 387 an seiner kugelkalottenförmigen Stirnseite mit einem starr mit ihm verbundenen Gewindebolzen versehen.

Nach dem Fräsen der Ringnut 1d kann die Fräslehre wieder entfernt werden, wonach der Knochen 1, d. h. das Hüftbein, das in der Figur 15 ersichtliche Loch besitzt, das sich aus der kugelkalottenförmigen Vertiefung 1b, der ein Gewinde aufweisenden Bohrung 1c und der Ringnut 1d zusammensetzt. In dieses Loch kann nun die Gelenkpfanne 5 eingeschoben werden, deren Schale 11 an der die Vertiefung 1b begrenzenden Fläche des Knochens anliegt und deren Verankerungsteil 17 satt in der Ringnut 1d sitzt. Die nach dem Einsetzen der Gelenkpfanne 5 frei bleibende Bohrung 1c wird dann im Laufe der Zeit wieder zuwachsen, wie es in der Figur 1 dargestellt ist.

Damit die in den Figuren 3, 10, 11 dargestellten Gelenkpfannen in einen Knochen eingesetzt werden können, wird dieser vorgängig bei der Operation analog bearbeitet, wie es für den Knochen 1 beschrieben wurde.

Wenn die in den Figuren 4, 6, 7, 8, 9 dargestellten Gelenkpfannen in einen Knochen eingesetzt werden sollen, fräst man in diesen ähnlich wie es in der Figur 12 dargestellt ist, zuerst die natürliche Gelenkpfanne aus, so dass eine mindestens annähernd halbkugelförmige Vertiefung zum Aufnehmen der Schale entsteht. Danach wird eine Fräslehre in die Vertiefung eingesetzt, die ähnlich wie die in der Figur 13 dargestellte Bohrlehre 383 einen in die Vertiefung passenden Körper und einen Handgriff aufweist, jedoch anstelle der koaxialen Führungsbohrung der Bohrlehre 383 eine Bohrung zum Führen eines Stirnfräsers aufweist. Diese Führungsbohrung ist derart gerichtet, dass eine Ringnut zum Aufnehmen des Verankerungsteils 77, 117, 177, 217 in den Knochen gefräst werden kann. Wenn auch noch das Einsetzen von dünnen schraubenartigen Verankerungsteilen 87, 127 vorgesehen ist, wird für diese im Knochen auch noch eine Bohrung oder Ringnut gebildet.

Bei denjenigen Varianten, bei denen die Verankerungsteile in den Knochen einzuschraubende Gewinde haben, müssen für diese, falls sie nicht selbstschneidend sind, natürlich in einem separaten Arbeitsgang noch Gewinde in den Knochen geschnitten werden.

Die beschriebenen künstlichen Gelenkpfannen sind insbesondere zur Bildung künstlicher Hüftgelenke bei Menschen oder auch bei Tieren, wie beispielsweise Hunden, vorgesehen.

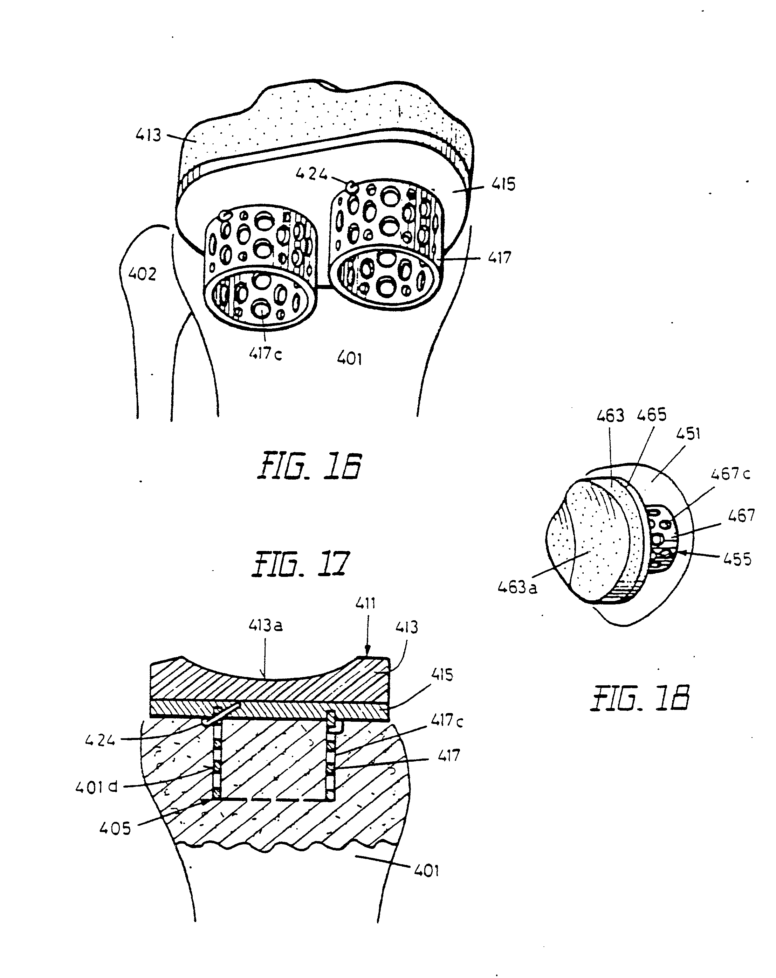

Es ist jedoch auch möglich die erfindungsgemässen Gelenkpfannen so anzupassen, wie beispielsweise in den Figuren 16 bis 20 dargestellt, dass sie als Pfannenkomponente anderer künstlicher Gelenke Verwendung finden können.

Die Figuren 16 und 17 zeigen den Knochen 401, nämlich eine Tibia, mit einer Gelenkpfanne 405 eines künstlichen Kniegelenkes mit einem plattenförmigen, metallischen Aussenteil 415, der proximal den Innenteil 413 und distal zwei Verankerungsteile 417 trägt. Der Innenteil 413 besteht aus der Kunststoff-Schale 411 mit einer konkaven Innenfläche 413a als Gleitpartner für eine nicht dargestellte konvexe Femurkomponente eines künstlichen Kniegelenkes. Der Aussenteil 415 weist an seiner distalen Seite zwei Ringnuten auf zur Fixierung von je einem Verankerungsteil 417 mittels Stiften 424, welche durch miteinander fluchtend angeordnete Bohrungen von Aussen- und Verankerungsteil gesteckt werden können. Die Verwendung zweier Verankerungsteile ergibt eine ausgezeichnete Rotationsstabilität der implantierten Tibia-Komponente. Der Verankerungsteil 417 kann entweder mit Perforationen 417c versehen sein und/oder gemäss den vorstehend beschriebenen Verankerungsteilen für die Hüftgelenkpfannen ausgebildet sein.

Die Gelenkpfanne 405 kann entweder vor der Operation zusammengesetzt werden — was den Vorteil einer limitierten Lagerhaltung von Einzelteilen für die verschiedenen Grössen besitzt — oder vorzugsweise den Chirurgen bereits in zusammengesetztem Zustand geliefert werden, wodurch Sterilisationsprobleme vermieden werden. Die Operationstechnik ist ähnlich derjenigen, welche für die Hüftgelenkpfannen beschrieben wurde, unter Anpassung an die spezifischen Erfordernisse und Instrumente der Kniegelenksimplantation.

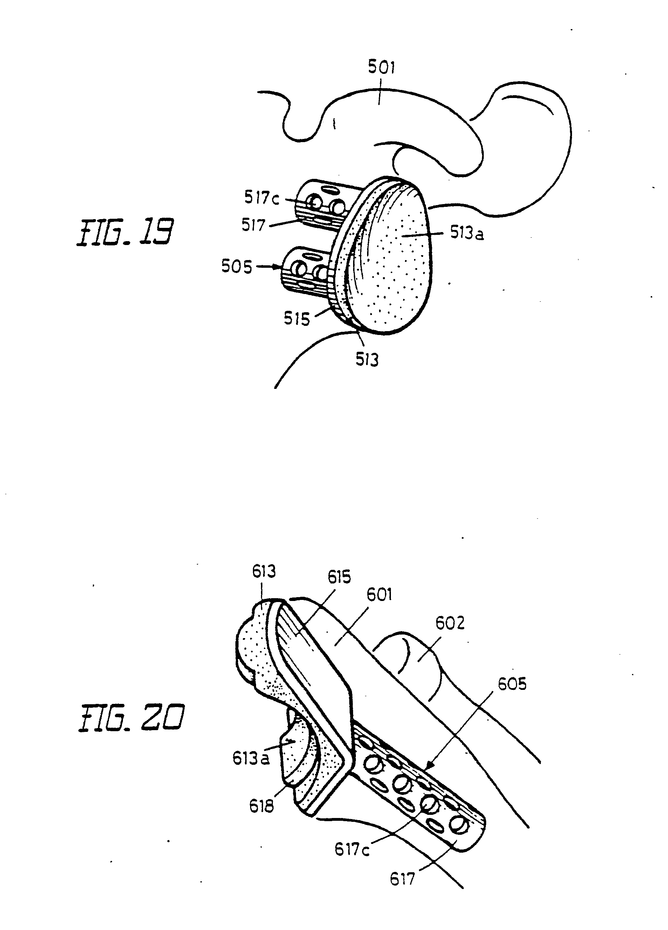

In Figur 18 ist eine erfindungsgemässe Patella-Komponente 455 einer Kniegelenkprothese dargestellt, welche im Knochen 451, nämlich eine Patella, implantiert ist. Die Patellakomponente 455 weist einen metallischen Aussenteil 465 auf, welcher den Innenteil 433 und den Verankerungsteil 467 trägt. Der Innenteil 433 besteht aus Kunststoff und hat eine konkave Innenfläche 463a. Der Verankerungsteil 467 kann gemäss den vorausgehend beschriebenen Varianten von Verankerungsteilen für künstliche Hüftgelenkspfannen ausgebildet sein.

In Figur 19 ist eine erfindungsgemässe Skapula-Komponente eines künstlichen Schultergelenkes dargestellt, mit einem metallischen Aussenteil 515 und einem Innenteil 513 mit einer konkaven Innenfläche 513a als Gleitpartner für eine nicht dargestellte konvexe Humerus-Komponente einer Schultergelenkprothese. Der Verankerungsteil 517 mit Perforationen 517c kann, ähnlich wie für die in Figur 17 dargestellte Tibiakomponente, am Aussenteil 515 befestigt werden.

Figur 20 zeigt schliesslich eine erfindungsgemässe Ulna-Komponente 605 eines künstlichen Ellbogengelenkes, welche im Knochen 601, nämlich einer Ulna, implantiert ist. Der gewinkelte Aussenteil 615 trägt wiederum einen Innenteil 613 mit einer konkaven Innenfläche 613a als Gleitpartner für eine nicht dargestellte konvexe Humerus-Komponente einer Ellbogenprothese.

Die Fixierung der Innenteile 413, 463, 513 und 613 an die Aussenteile 415, 465, 515 und 615 kann durch beliebige, bekannte Befestigungsvorrichtungen für zwei im wesentlichen planare Komponenten erfolgen, vorzugsweise durch einen lösbaren Befestigungsmechanismus im Hinblick auf einen später notwendig werdenden Austausch einzelner Komponenten der implantierten Gelenkpfanne.

Bei den in den Figuren 1 bis 11 und 16 bis 20 dargestellten Varianten sind die Verankerungsteile derart an den Schalen, bzw. an den Aussenteilen der Schalen gehalten, dass sie mindestens von den Schalen-Aussenflächen weggerichtete, zu ihren Achsen parallele Zugkräfte auf die Schale übertragen können. Es wäre jedoch auch noch möglich, eine künstliche Gelenkpfanne mit mindestens einem hohlzylindrischen Verankerungsteil herzustellen, der nicht zugfest an der Schale angreift, sondern lediglich bei in einen Knochen eingesetzter Gelenkpfanne mit seinem einen Ende an der Schale anliegt, und zwar vorzugsweise an deren Aussenfläche. Das andere Ende des Verankerungsteils würde dann wie bei allen anderen beschriebenen und dargestellten Ausführungsvarianten der erfindungsgemässen Gelenkpfanne von der Schalen-Aussenfläche wegragen. Bei einer derartigen Gelenkpfanne könnte der Verankerungsteil die Schale abstützen, wobei mindestens solche Druckkräfte von der Schale auf die Hülse und umgekehrt übertragen werden können, die ungefähr parallel zur Achse des Verankerungsteils gerichtet sind. Dabei würden bei der Berührungsstelle, bei der der Verankerungsteil an der Schale anliegt, zweckmässigerweise Stütz- und Führungsmittel vorgesehen, die den Verankerungsteil bezüglich der Schale seitlich abstützen und auch die Übertragung von quer zur Achse des Verankerungsteils gerichteten Druckkräfte ermöglichen. Beispielsweise könnten bei der in der Figur 7 dargestellten Gelenkpfanne 165 der Verbindungsteil 179 und die Schraube 181 weggelassen werden. Dabei würde dann die Ringnut 175a in Zusammenwirkung mit dem in sie hineinragenden Rand des Verankerungsteils die erwähnten Stütz- und Führungsmittel bilden. Die Schale könnte statt mit der erwähnten Ringnut oder zusätzlich zu dieser zur Bildung der genannten Stütz- und Führungsmittel mit einem über ihre restliche Aussenfläche vorstehenden, den Verankerungsteil zentrierenden Vorsprung versehen sein, der satt oder mit geringem radialen Spiel in das Verankerungsteil-Ende hineinragt oder dieses umschliesst und beispielsweise einen ringförmigen Kragen bildet. Im übrigen könnte die Schale bei diesen Varianten statt aus einem Innenteil aus Kunststoff und einem Aussenteil aus Metall ohne weiteres ausschliesslich aus einem einstückigen, beispielsweise aus Kunststoff bestehenden Körper gebildet sein.

**Patentansprüche**

1. Ohne Knochenzement einzusetzende künstliche Gelenkpfanne, insbesondere für ein Hüftgelenk, mit einer eine konkave, vorzugsweise kugelkalottenförmige, Innenfläche (13a) aufweisenden Schale (11) und mindestens einem zum Verankern in einem Knochen (1) bestimmten Verankerungsteil (17), der mindestens bei in einen Knochen (1) eingesetzter Gelenkpfanne an seinem einen Ende von der Schale (11) gehalten ist und/oder an dieser anliegt und der mindestens am von der konvexen Schalen-Aussenfläche wegverlaufenden Abschnitt im allgemeinen kreiszylinderförmig ist, dadurch gekennzeichnet, dass der Verankerungsteil (17) mindestens zum grössten Teil hohl und an seinem der Schale (11) abgewandten freien Ende offen ist.

2. Gelenkpfanne nach Anspruch 1, dadurch gekennzeichnet, dass der Verankerungsteil (17) durchgehend als hohler Kreiszylinder ausgebildet ist.

3. Gelenkpfanne nach Anspruch 1 oder 2, dadurch gekennzeichnet, dass die Achse (23) des Verankerungsteils (17) durch das Zentrum (21) der von der Schalen-Innenfläche (13a) aufgespannten Kugel verläuft.

4. Gelenkpfanne nach Anspruch 3, dadurch gekennzeichnet, dass die Schale (11) im allgemeinen rotationssymmetrisch zur genannten Achse (23) des Verankerungsteiles (17) ist.

5. Gelenkpfanne nach einem der Ansprüche 1 bis 4, dadurch gekennzeichnet, dass der von der Aussenfläche des Verankerungsteils (17) aufgespannte Zylinder die Innenfläche (13a) der Schale (11) umschliesst, wobei der Verankerungsteil (17) vorzugsweise in der Nähe des Randes der Schale (11) mit dieser verbunden ist oder an dieser anliegt und wobei der Aussendurchmesser des Verankerungsteiles (17) vorzugsweise ungefähr gleich dem Durchmesser der von der Aussenfläche der Schale (11) aufgespannten Kugelfläche ist.

6. Gelenkpfanne nach einem der Ansprüche 1 bis 5, dadurch gekennzeichnet, dass das der Schale (11) abgewandte, freie Ende des Verankerungsteiles (17) in axialer Richtung höchstens 10 % des Kugel-Radius der kugelkalottenförmigen Innenfläche (13a) der Schale (11) über diese herausragt und sich der Verankerungsteil (17) vorzugsweise von seinem mit der Schale (11) verbundenen oder an dieser anliegenden Ende weg in axialer Richtung höchstens bis zur Stelle erstreckt, bei der seine Achse (23) die Aussenfläche der Schale (11) schneidet, und also vorzugsweise überhaupt nicht über die Schale (11) herausragt.

7. Gelenkpfanne nach einem der Ansprüche 1 bis 6, dadurch gekennzeichnet, dass der Innendurchmesser des Verankerungsteils (17) mindestens 20 %, vorzugsweise mindestens ein Drittel und beispielsweise mindestens zwei Drittel des Durchmessers der von der kugelkalottenförmigen Schalen-Innenfläche (13a) definierten Kugel beträgt oder eventuell sogar mindestens gleich diesem Durchmesser ist.

8. Gelenkpfanne nach einem der Ansprüche 1 bis 7, dadurch gekennzeichnet, dass der Verankerungsteil perforiert ist und/oder an der Aussenseite mindestens eine Erhöhung, beispielsweise mindestens eine ein Gewinde bildende oder ringförmige Rippe oder über den Umfang verteilte, axiale Rippen, aufweist.

9. Gelenkpfanne nach einem der Ansprüche 1 bis 3 oder 5 bis 8, dadurch gekennzeichnet, dass der Verankerungsteil (77) asymmetrisch zur Aussenfläche der Schale (71) an dieser angeordnet ist und eine vorzugsweise vorhandene Rotationssymmetrieachse (83) der Schale (71) mit der Achse (85) des Verankerungsteils (77) einen Winkel bildet, der beispielsweise 20° bis 45° beträgt.

10. Gelenkpfanne nach einem der Ansprüche 1 bis 9, dadurch gekennzeichnet, dass die Schale (11) einen die Innenfläche (13a) bildenden Innenteil (13) und einen vorzugsweise mit dem Verankerungsteil (17) verbundenen, beispielsweise mit diesem zusammenhängenden Aussenteil (15) aufweist und dass der Innenteil (13) und der Aussenteil (15) durch separate Körper gebildet und miteinander verbunden sind, wobei der Innen-und der Aussenteil vorzugsweise durch ineinander einrastbare Rastmittel (13d, 17b) zusammengehalten sind und der Innenteil (13) vorzugsweise auch dann mit dem Aussenteil (15) verbindbar sowie unter eventueller Beschädigung des Innenteils (13) vom Aussenteil (15) trennbar ist, wenn der Aussenteil (15) und der Verankerungsteil (17) in einem Knochen (1) sitzen.

11. Gelenkpfanne nach einem der Ansprüche 1 bis 10, dadurch gekennzeichnet, dass der Verankerungsteil (17) und mindestens ein zum Anliegen am Knochen (1) bestimmter Aussenteil (15) der Schale (11) aus Metall bestehen, wobei ein die Innenfläche (13a) bildender Innenteil (13) der Schale (11) vorzugsweise aus einem nichtmetallischen Material, beispielsweise Kunststoff, besteht.

12. Gelenkpfanne nach einem der Ansprüche 1 bis 11, dadurch gekennzeichnet, dass der Verankerungsteil (17) oder mindestens ein Verankerungsteil (77) mit der Schale oder mindestens mit einem zum Anliegen an einem Knochen (1) bestimmten Aussenteil (15, 75) der Schale (11, 71) starr und unlösbar verbunden ist.

13. Gelenkpfanne nach einem der Ansprüche 1 bis 11, dadurch gekennzeichnet, dass der Verankerungsteil (117, 177, 317) lösbar mit der Schale (111, 171, 211) verbunden ist, wobei der Verankerungsteil beispielsweise um seine Achse (125) drehbar an der Schale (111) gehalten oder durch Verschraubungsmittel (179, 181) mit der Schale (171) verbunden ist.

14. Gelenkpfanne nach Anspruch 13, dadurch gekennzeichnet, dass der Verankerungsteil (117, 177, 317) mit einem zum Einschrauben in den Knochen bestimmten Gewinde versehen ist.

15. Gelenkpfanne nach einem der Ansprüche 1 bis 14, dadurch gekennzeichnet, dass die Wandstärke des Mantels des Verankerungsteils (17) höchstens 10 %, vorzugsweise höchstens 5 % des Innendurchmessers des Verankerungsteils beträgt.

16. Gelenkpfanne nach einem der Ansprüche 1 bis 15, dadurch gekennzeichnet, dass der Aussenteil (415) im allgemeinen eben, beispielsweise als Plateau, ausgebildet ist.

17. Gelenkpfanne nach einem der Ansprüche 1 bis 16, dadurch gekennzeichnet, dass der Aussenteil (415) mit mindestens zwei Verankerungsteilen (417) starr oder lösbar verbunden ist, vorzugsweise mit je einem Verankerungsteil (417) auf der medialen und lateralen Seite des Aussenteils (415).

18. Verwendung der Gelenkpfanne nach einem der Ansprüche 1 bis 17 als Tibia-Komponente einer Kniegelenkprothese, vorzugsweise mit einer den natürlichen tibialen Kondylenoberflächen nachgebildeten konkaven Innenfläche (463a) als Gleitpartner für eine konvexe Femur-Komponente einer Kniegelenkprothese.

19. Verwendung der Gelenkpfanne nach einem der Ansprüche 1 bis 17 als Patella-Komponente einer Kniegelenkprothese.

20. Verwendung der Gelenkpfanne nach einem der Ansprüche 1 bis 17 als Skapula-Komponente einer Schultergelenkprothese.

21. Verwendung der Gelenkpfanne nach einem der Ansprüche 1 bis 17 als Ulna-Komponente einer Ellbogengelenkprothese mit einer konkaven Innenfläche (613a) als Gleitpartner für eine mit einer korrespondierenden konvexen Innenfläche versehenen Humerus-Komponente einer Ellbogengelenkprothese.

### Claims

1. Artificial joint socket for cementless implantation, particularly for a hip joint, comprising a cup (11) with an interior concave surface (13a), preferably in the form of a spherical cap, and at least one anchoring part (17) intended for anchorage in a bone (1), which at least in the implanted state is at one of its ends held by and/or adjacent to the cup (11) and of which at least that section which extends away from the convex exterior surface of the cup (11) is generally cylindrical, characterised in that the anchoring part (17) is at least for the major part hollow, and open at the free end opposite to the cup (11).

2 Artificial joint socket according to claim 1, characterised in that the anchoring part (17) is designed continuously as a hollow circular cylinder.

3. Artificial joint socket according to claim 1 or 2, characterised in that the rotational axis of symmetry (23) of the anchoring part (17) is passing through the center (21) of the sphere formed by the interior surface (13a).

4. Artificial joint socket according to claim 3, characterised in that the cup (11) is in general rotational-symmetrical with regard to the axis (23) of the anchoring part (17).

5. Artificial joint socket according to one of the claims 1 to 4, characterised in that the cylinder formed by the exterior surface of the anchoring part (17) is encompassing the interior surface (13a) of the cup (11), whereby the anchoring part (17) is connected with or adjacent to the cup (11), preferably in the proximity of the border of the cup (11), and whereby the exterior diameter of the anchoring part (17) corresponds preferably approximately to the diameter of the sphere formed by the exterior surface of the cup (11).

6. Artificial joint socket according to one of the claims 1 to 5, characterised in that the free end of the anchoring part (17) opposite to the cup (11) is projecting over the cup (11) in axial direction by the amount of 10 % at the most of the radius of the sphere formed by the interior surface (13a) of the cup (11) in the form of a spherical cap and that the anchoring part (17) preferably is extending in axial direction from its end connected with or adjacent to the cup (11) at the most to the point where its axis (23) is intersecting the exterior surface of the cup (11) and therefore preferably does not project over the cup (11) altogether.

7. Artificial joint socket according to one of the claims 1 to 6, characterised in that the interior diameter of the anchoring part (17) is equivalent to at least 20 %, preferably at least to one third and for example to at least two thirds of the diameter of the sphere formed by the interior surface (13a) in the form of a spherical cap, or possibly at least equivalent to that diameter.

8. Artificial joint socket according to one of the claims 1 to 7, characterised in that the anchoring part (17) is perforated and/or comprises at its external surface at least one projection, for example at least one threaded or annular rib, or axial ribs distributed over the circumference.

9. Artificial joint socket according to one of the claims 1 to 3 or 5 to 8, characterised in that the anchoring part (77) is disposed asymmetrically to the exterior surface of the cup (71) and that a preferably existing rotational axis of symmetry (83) of the cup (71) is intersecting the axis (85) of the anchoring part (77) at an angle, preferably between 20° and 45°.

10. Artificial joint socket according to one of the claims 1 to 9, characterised in that the cup (11) comprises an interior part (13) forming the interior surface (13a) and an exterior part (15) preferably connected to the anchoring part (17), for example fixed to it and that the interior part (13) and the exterior part (15) are formed by separate bodies connected to each other, whereby the interior part (13) and the exterior part (15) are preferably held together by interlocking means (13d, 17b) and whereby the interior part (13) is preferably interlockable with and possibly under deterioration unlockable from the exterior part (15) even when the exterior part (15) and the anchoring part (17) are implanted in a bone (1).

11. Artificial joint socket according to one of the claims 1 to 10, characterised in that the anchoring part (17) and at least an exterior part (15) of the cup (11) destined to be adjacent to the bone (1) are made from metal, whereby an interior part (13) of the cup (11) forming the interior surface (13a) is made preferably from a non-metallic material, for example plastics.

12. Artificial joint socket according to one of the claims 1 to 11, characterised in that the anchoring part (17) or at least an anchoring part (77) is rigidly and unseparably connected to the cup (11) or at least to an exterior part (15, 75) of the cup (11, 71) destined to be adjacent to the bone (1).

13. Artificial joint socket according to one of the claims 1 to 12, characterised in that the anchoring part (117, 177, 317) is detachably connected to the cup (111, 171, 211), whereby the anchoring part is held preferably rotatably around its axis (125) at the cup (111) or is connected by connecting means (179, 181) to the cup (171).

14. Artificial joint socket according to claim 13, characterised in that the anchoring part (117, 177, 317) comprises a thread destined to be screwed into the bone (1).

15. Artificial joint socket according to one of the claims 1 to 14, characterised in that the wall thickness of the convex surface of the anchoring part (17) corresponds to 10 % at the most and preferably 5 % at the most of the interior diameter of the anchoring part.

16. Artificial joint socket according to one of the claims 1 to 15, characterised in that the exterior part (15, 415) is generally planar, e. g. in the form of a plateau.

17. Artificial joint socket according to one of the claims 1 to 16, characterised in that the exterior part (15, 415) is connected rigidly or removably with at least two anchoring parts (417), preferably with an anchoring part (417) each at the lateral and medial side of the exterior part (15, 415).

18. Use of the artificial joint socket according to one of the claims 1 to 17 as a tibial component of a knee joint prosthesis, preferably with an interior concave surface (463a) adapted to the natural tibial condyle surfaces and destined for gliding engagement with a convex femoral component of a knee joint prosthesis.

19. Use of the artificial joint socket according to one of the claims 1 to 17 as a patellar component of a knee joint·prosthesis.

20. Use of the artificial joint socket according to one of the claims 1 to 17 as a glenoid component of a shoulder joint prosthesis.

21. Use of the artificial joint socket according to one of the claims 1 to 17 as an ulnar component of an elbow joint prosthesis with an interior concave surface (613a) adapted for gliding engagement with the convex surface of a humeral component of an elbow joint prosthesis.


**Revendications**

1. Cupule artificielle pour articulation pour implantation exempte de ciment, en particulier pour l'articulation de la hanche, comportant une cupule (11) avec une surface intérieure concave (13a), préférablement en forme de calotte sphérique, et au moins une partie d'ancrage (17) destinée à être ancrée dans l'os (1), laquelle est au moins dans l'état implanté tenue par/ou adjacent à la cupule (11) à une des ses extrémités et dont au moins la section s'éloignant de la surface extérieure convexe de la cupule (11) est en général cylindrique, caractérisée en ce que la partie d'ancrage (17) est au moins dans sa majorité creuse et ouverte à son extrémité libre opposée à la cupule (11).

2. Cupule artificielle pour articulation selon la revendication 1, caractérisée en ce que la partie d'ancrage (17) est réalisée de manière continue en forme de cylindre circulaire creux.

3. Cupule artificielle pour articulation selon la revendication 1 ou 2, caractérisée en ce que l'axe de symétrie (23) de la partie d'ancrage (17) passe à travers le centre (21) de la sphère formée par la surface intérieure (13a).

4. Cupule artificielle pour articulation selon la revendication 3, caractérisée en ce que la cupule (11) est en général symétrique par rapport à l'axe de rotation (23) de la partie d'ancrage (17).

5. Cupule artificielle pour articulation selon l'une des revendications 1 à 4, caractérisée en ce que le cylindre formé par la surface extérieure de la partie d'ancrage (17) englobe la surface intérieure (13a) de la cupule (11), en ce que la partie d'ancrage (17) est combinée avec ou adjacente à la cupule (11), préférablement en proximité du bord de la cupule (11), et en ce que le diamètre extérieur de la partie d'ancrage (17) correspond préférablement de manière approximative au diamètre de la sphère formée par la surface extérieure de la cupule (11).

6. Cupule artificielle pour articulation selon l'une des revendications 1 à 5, caractérisée en ce que l'extrémité libre de la partie d'ancrage (17) opposée à la cupule (11) fait saillie sur la cupule (17) en direction axiale par le 10 % au maximum du rayon de la sphère formée par la surface intérieure (13a) de la cupule (11) en forme de calotte sphérique et en ce que la partie d'ancrage (17) préférablement fait saillie en direction axiale de son extrémité combinée avec ou adjacente à la cupule (11) au maximum jusqu'au point où son axe (23) intersecte la surface extérieure de la cupule (11) et préférablement donc ne fait pas saillie sur la cupule (11) point du tout.

7. Cupule artificielle pour articulation selon l'une des revendications 1 à 6, caractérisée en ce que le diamètre intérieur de la partie d'ancrage (17) est équivalent au moins au 20 %, préférablement au moins à un tiers et par exemple au moins à deux tiers du diamètre de la sphère formée par la surface intérieure (13a) en forme de calotte sphérique, ou qu'il est équivalent éventuellement au moins à ce diamètre.

8. Cupule artificielle pour articulation selon l'une des revendications 1 à 7, caractérisée en ce que la partie d'ancrage (17) est perforée et/ou comporte à sa surface externe au moins une saillie, par exemple au moins une nervure annulaire ou formant un filetage, ou nervures axiales distribuées sur la circonférence.

9. Cupule artificielle pour articulation selon l'une des revendications 1 à 3 ou 5 à 8, caractérisée en ce que la partie d'ancrage (77) est disposée de manière asymétrique par rapport à la surface extérieure de la cupule (71) et qu'un axe rotatif de symétrie (83) de la cupule (71), préférablement existant, intersecte l'axe (85) de la partie d'ancrage (77) avec un angle, préférablement entre 20° et 45°.

10. Cupule artificielle pour articulation selon l'une des revendications 1 à 9, caractérisée en ce que la cupule (11) comporte une partie intérieure (13) formant la surface intérieure (13a) et une partie extérieure (15) préférablement combinée avec la partie d'ancrage (17), par exemple fixé à celle-ci et que la partie intérieure (13) et la partie extérieure (15) sont matérialisées par des corps séparés combinés l'un avec l'autre, la partie intérieure (13) et la partie extérieure (15) étant préférablement tenues ensemble par des moyens d'encliquetage (13d, 17b) prenant l'encoche l'un dans l'autre, et la partie intérieure (13) étant préférablement combinable avec et — subissant éventuellement un endommagement — détachable de la partie extérieure (15) même si la partie extérieure (15) et la partie d'ancrage (17) sont implantées dans l'os (1).

11. Cupule artificielle pour articulation selon l'une des revendications 1 à 10, caractérisée en ce que la partie d'ancrage (17) et au moins une partie extérieure (15) de la cupule (11) destinée à être adjacent à l'os (1) sont en métal, une partie intérieure (13) de la cupule (11) formant la surface intérieure (13a) consistant préférablement en une matière non métallique, par exemple en une matière plastique.

12. Cupule artificielle pour articulation selon l'une des revendications 1 à 11, caractérisée en ce que la partie d'ancrage (17) ou au moins une partie d'ancrage (77) est combinée de manière rigide et inséparable à la cupule (11) ou au moins à une partie extérieure (15, 75) de la cupule (11, 71) destinée à être adjacent à l'os (1).

13. Cupule artificielle pour articulation selon l'une des revendications 1 à 12, caractérisée en ce que la partie d'ancrage (117, 177, 317) est combinée de manière détachable à la cupule (111, 171, 211), la partie d'ancrage (117, 177, 317) étant tenue à la cupule (111) préférablement de manière rotative autour de son axe (125) ou étant combinée à la cupule (171) par moyens de vissage (179, 181).

14. Cupule artificielle pour articulation selon l'une des revendications 1 à 13, caractérisée en ce que la partie d'ancrage (117, 177, 317) est munie d'un filetage destiné à être vissé dans l'os (1).

15. Cupule artificielle pour articulation selon l'une des revendications 1 à 14, caractérisée en ce que l'épaisseur de la surface convexe de la partie d'ancrage (17) corresponde au 10 % au maximum et préférablement au 5 % au maximum du diamètre intérieur de la partie d'ancrage (17).

16. Cupule artificielle pour articulation selon l'une des revendications 1 à 15, caractérisée en ce que la partie extérieure (15, 415) est en général plane, par exemple en forme de plateau.

17. Cupule artificielle pour articulation selon l'une des revendications 1 à 16, caractérisée en ce que la partie extérieure (15, 415) est combinée de manière rigide ou amovible avec au moins deux parties d'ancrage (417), préférablement avec une partie d'ancrage (417) chacune sur le côté latéral et médial de la partie extérieure (15, 415).

18. Utilisation de la cupule artificielle pour articulation selon l'une des revendications 1 à 17, comme composant tibial d'une endo-prothèse de genou, préférablement avec une surface intérieure concave (463a) correspondante aux surfaces des condyles tibiaux naturels comme pièce opposé de glissage pour un composant fémoral convexe d'une endo-prothèse de genou.

19. Utilisation de la cupule artificielle pour articulation selon l'une des revendications 1 à 17, comme composant patellaire d'une endo-prothèse de genou.

20. Utilisation de la cupule artificielle pour articulation selon l'une des revendications 1 à 17, comme composant glénoïde d'une endo-prothèse d'épaule.

21. Utilisation de la cupule artificielle pour articulation selon l'une des revendications 1 à 17, comme composant cubital d'une endo-prothèse de coude avec une surface intérieure concave (613a) comme pièce opposé de glissage pour un composant huméral-muni d'une surface convexe correspondante.

FIG. 1

FIG. 2

*FIG. 3*

*FIG. 4*

*FIG. 5*

FIG. 6

FIG. 7

FIG. 8

225

217

215c

215a

215b

215

IX

FIG. 9

215c

215b

215c

215a

215

215b

FIG. 10

317

305

315d

315c

313c

315b

313b

313

FIG. 11

311

315

317

305

317a

315c

313

313b

315b

4

FIG. 12

381
1b
1a
1

FIG. 13

1
1c
1b
385
383

FIG. 14

1
1c
1b
389
1d
387
391

FIG. 15

1
1c
1b
1d

FIG. 16

FIG. 17

FIG. 18

501

517c
517
505

513a

515

513

_FIG. 19_

613    615    601    602

605

_FIG. 20_

613a

618

617c

617